# EUROPEAN PATENT SPECIFICATION

(11) **EP 1 811 979 B1**
(45) Date of publication and mention of the grant of the patent: **05.11.2008**
(21) Application number: 05786942.2
(22) Date of filing: 27.09.2005
(51) Int. Cl.: A61K 9/50, A61K 31/4422

(54) **MICROCAPSULES COMPRISING A METHYLXANTHINE AND A CORTICOSTEROID**
MIKROKAPSELN MIT EINEM METHYLXANTHIN UND EINEM KORTIKOSTEROID
MICROCAPSULES COMPRENANT METHYLXANTHINE ET UN CORTICOSTEROIDE

(30) Priority: 27.09.2004 US 612784 P; 27.09.2004 US 612785 P; 27.09.2004 US 612786 P
(43) Date of publication of application: 01.08.2007
(73) Proprietor: Sigmoid Pharma Limited, Dublin 9 (IE)
(72) Inventor: MOODLEY, Joey, Athlone, County Westmeath (IE); COULTER, Ivan, Dublin 2 (IE)
(74) Representative: O'Brien, John Augustine
(86) International application number: PCT/IE2005/000106
(87) International publication number: WO 2006/035418

(56) References cited:
- EP-A- 0 670 163
- EP-A- 0 778 083
- EP-A- 0 922 451
- US-A1- 2003 078 194

## Description

### Field of the Invention

This invention relates to novel combination approaches to enhance therapeutic benefit or active bioavailability. In some disease treatment instances, more than one active is recommended, either under label or off-label, to increase the overall drug effectiveness or efficacy. In other instances, a combination of actives may enhance the bioavailability of one or all actives.

### Background of the Invention

Drug combination approaches are gaining in popularity. The main drivers are a recognition that such approaches often lead to a better therapeutic outcome and a greater understanding both of the molecular events leading to disease as well as molecular intervention strategies. While a number of combination products are in clinical use, mainly in the cardiovascular field, other desirable combinations have not been possible due to formulation incompatibilities, such as, for example, the fact that lipid-based and water-based formulations do not mix. This invention is directed to formulations which will enable the development of heretofore incompatible drugs being administered as a single pill.

A major issue affecting drug effectiveness is the requirement for high drug dosage resulting in much waste and often toxicities. The underlying reasons necessitating such high dosage include poor drug absorption from the intestine, drug metabolism by enzymes of the cytochrome P450 class, including the various 3A subset family, that reside in the cells lining the intestine and in the liver, drug degradation by low pH in the stomach and enzymes in the small intestine, including protease and peptidases, as well as various cellular pump systems that cause efflux of drugs from the cells, including PgP efflux pumps. Further issues relate to the development of resistance following un-going drug administration. The underlying cause for such resistance often is an inbuilt molecular protective mechanism such as that controlled by histone deacetylase in the case of COPD resistance to corticosteroids.

Co-administering a drug with poor oral bioavailability or stability with metabolism, including cytochrome P450 3A, inhibitors, degradative enzyme inhibitors, efflux inhibitors increase drug bioavailability and stability, thereby increasing drug dosing efficiency, reducing dose size and frequency as well as ensuring that the active drug is less toxic and thus better tolerated.

A further driver for novel drug combinations is that the aetiology of many diseases steams from multiple molecular pathway dysfunction. The result is that while individual drugs, targeting individual pathways, improve the symptoms a number of the underlying causes are left untreated, resulting in a chronic deterioration of the patient's health. Combination therapies, targeting a number of the aetiological malfunctions, have the potential to lead to improved disease management and patient health and wellbeing. Examples of diseases stemming from multiple aetiological are numerous, including cancer, cardiovascular and heart disease, central nervous system disorder, immunological conditions and diabetes.

EP 0670163 discloses a therapeutic agent comprising a combination of pentoxifylline and methylprednisolone for treating granulomatous and fibrotic lung disease.

A major impedance to combination therapy develop stems from the incompatibility of different drug formulations. Generally speaking water soluble drugs are not compatible with lipid or oil-soluble drugs. Additionally, many labile drugs arc effective only for short time periods, often less than 6 hours. As such, patients are required to take multiple pills, often several times a day, a major inconvenience..

Drug formulation approaches are therefore required that address solubility, instability, efficacy, safety, tolerability, convenience and compliance issues.

Drugs that are poorly water soluble must be formulated in a way that improves their solubility and hence their bioavailability. In general, drug that is in solution or suspension when administered by the oral route is rapidly and frequently instantaneously absorbed from the gastrointestinal tract resulting in a fast therapeutic action. However, in many cases it is desirable to control the rate of absorption following oral administration in order to achieve the desired plasma profile or prolongation of action or to avoid any side effects of the drug.

Numerous processes and formulations exist to enhance the solubility of poorly soluble drug compounds. Solubilisation of such drugs in solvents, oils, emulsions and microemulsions are well known to those skilled in the art and have been used to deliver such drugs orally. Such formulations are then encapsulated in soft gelatine capsules for oral administration. Soft gelatine encapsulation is a specialised process. Soft gelatine capsules do not lend themselves easily to further processing such as the addition of delayed or sustained release coatings.

There is also a need for a formulation which will allow the delivery of a drug to the optimum site of absorption/action in the gastrointestinal tract.

Also, in some cases there is a need for increased time of residence or delivery to certain locations along the GIT, either to optimise systemic bioavailability or localised activity.

Methylxanthines, especially in this case Theophylline, pentoxifylline and A802715 are useful medicines frequently used, due to bronchodilatory effects, as an agent for treating symptoms of bronchial asthma. As known in the art the effective blood levels is about 10-20 mg/L. If the concentration in the blood exceeds 20 mg/L it has been seen to have serious side effects on the cardiovascular and nervous systems (Barnes, PJ. Current Treatments for asthma: Promises and Limitations. Chest 1997; 111:17S-22S). Furthermore difference in blood levels among individuals, cardiac insufficiency, liver and kidney disease, age, smoking etc. also have effects. Recently, it has been suggested that, at lower concentrations of between 5-10 mg/L that Theophylline may have an anti-inflammatory and immunostimulatory effect (Barnes PJ, Pauwels RA. Theophylline in asthma: time for a reappraisal? Eur Respir J 1994; 7: 579-591). Theophylline has a short biological half life <6 hours for adults, therefore it is necessary for multiple dosing (QID) to achieve effective blood levels have been considered. To address this issue of multiple dosing, numerous sustained release type formulations have been developed. However, these sustained release formulation suffer from a number of problems. Principal amongst these is the need for a zero- or pseudo-zero-order release profile in a format that is compatible with other often co-administered drugs, administration of which benefit from sustained release.

Oral administration of corticosteroids is widely prescribed to reduce inflammation in both Asthma and Chronic Obstructive Pulmonary Disorder (COPD) as well as for other inflammation-related diseases. While effective in a majority of asthmatics and a large proportion of COPD patients, long term administration of high corticosteroid doses has been associated with a number of side effects, including osteoporosis, hypertension and weight gain. As a result of COPD-related cellular oxidative stress a large proportion of COPD patients have developed resistance to corticosteroid therapies (Kirsten DK, Wenger RE, Jorres RA, Magnussen H. Effects of theophylline withdrawal in severe chronic obstructive pulmonary disease. Chest 1993; 104: 1101-1107).

To optimise disease management, oral administration of numerous cardiovascular drugs is common place. Various cardiovascular drug combinations are prescribed to treat heart and vascular disease and to prevent initial or further disease related incidences. Additionally, a number of cardiovascular drugs have shown potential and clinical effectiveness in the treatment or prevention of symptoms or events that arise or give rise to other diseases such as diabetes related morbidities and mortality.

A number of interventive strategies to avert an escalating crisis of heart attacks and strokes is ongoing. Gaining prominence as an interventive and prophylactic as well as therapeutic approach is the concept of a single formulation of six medications given to those most at risk. This debate was strengthened by the publication in British Medical Journal (BMJ 2003; 326: 1419 Wald and Law) of a proposal by British medical scientists that all heart patients and all those aged 55 should be targeted to receive a single formulation of six medications, the so-called 'polypill'..

The 'polypill', proposed by Dr Nicolas Wald and Dr Malcolm Law of the University of London, comprises a statin or simvastatin to lower blood LDL-cholesterol, three blood-pressure lowering drugs at half dose (e.g., a thiazide diuretic, a beta-blocker and an angiotensin converting enzyme (ACE) inhibitor, folic acid to reduce levels of homocysteine and subsequent atherosclerosis, and aspirin, as an anticoagulant.

A difficulty associated with development of a single pill containing up to six drug formulations is the incompatibility of the individual drug formulations, incompatibilities such as oil versus water soluble, long versus short half-life, differential circadian-related illness drug chronotherapy requirements. A multicomponent aggregator technology is required to ensure that a single unit 'polypill' is made possible.

In addition to the intended pharmaceutical action, several cardiovascular drug classes have been shown to have a range of activities. These include, for example, Angiotensin II inhibitors attenuate some of the actions that Angiotensin II has been shown to foster, including inflammation, plaque formation, fibrosis and excessive growth of arterial walls and the left ventricle.

As well as Angiotensin II Inhibitors, Animal and clinical studies suggest that ACE inhibitors hinder many of these damaging processes. ACE inhibitors also may benefit people with cardiovascular disease by encouraging new blood vessels to sprout. One ACE inhibitor, quinapril, was recently shown to do this in animal studies. By lessening inflammation and fibrosis, ACE inhibitors are thought to stall the progress of kidney disease. ACE inhibitors can also boost blood flow to the pancreas, reduce insulin clearance from the liver and hamper inflammation.

Additionally, many of the ACE inhibitors are now approved for use in congestive heart failure (The CONSENSUS Trial Study Group. Effects of enalapril on mortality in severe congestive heart failure. Results of the Cooperative North Scandinavian Enalapril Survival Study (CONSENSUS). N Engl J Med 1987;316:1429-35.).

In a range of clinical studies, ACE Inhibitors have been shown to have positive effects on diabetes onset and progression. Following onset of diabetes, however, most studies seem to suggest that ACE inhibitors can help in several ways:
• The drugs cut in half the incidence of cardiovascular events and produce modest reductions in blood pressure.
• ACE inhibitors lower the risk of developing or experiencing a progression of kidney disease by one-quarter, a degree not attainable by other hypertensive drugs-except for angiotensin receptor blockers
• The drugs slow the progression of retinopathy in patients with normal blood pressure levels.

The American Diabetes Association recommends ACE inhibitors for diabetics with microalbuminuria. The organization also recommends the drugs for diabetics over 55 with hypertension, and for diabetics who don't have hypertension but have another risk factor for cardiovascular disease. It is a rare diabetes patient who doesn't meet one of these criteria.

Likewise, statins have been shown to a range of actions such as anti-inflammatory and prevent the growth of Alzheimer's Disease -associated plaque formation (Association between statin use and Alzheimer's disease. Zamrini et al.; Neuroepidemiology. 2004 Jan-Apr; 23(1-2):94-8.).

A recent study, reported in the New England Journal of Medicine (N Engl J Med. 2005 Jul 7;353(1):85-6.), suggests that hydrourea, if combined with a small dose of aspirin is effective in the treatment and prevention of thrombocythemia and is more effective in preventing serious bleeding. Thrombocythemia occurs when too many blood-clotting structures known as platelets are produced in the bone marrow. It can affect other types of blood cells and can cause a host of problems including chest pain, bleeding, and leg and lung clots. It usually strikes in middle age, affecting 1 in 30,000 people. In a Journal editorial, Tiziano Barbui and Guido Finazzi of Ospedali Riuniti in Bergamo, Italy, said "for now, hydroxyurea plus aspirin should be the standard of treatment for patients with essential thrombocythemia" whose disease makes them vulnerable to blood clots.

Even following entry into tumour cells, anticancer agents, often plagued by low solubility and/or poor permability, are susceptible to ejection from the cell through the action of efflux pumps: A number of efflux pump inhibitors has been identified, including cyclosporine A (Efficacy of novel P-glycoprotein inhibitors to increase the oral uptake of paclitaxel in mice. Bardelmeijer et al., Invest New Drugs. 2004 Aug;22(3):219-29.), cimetidine (Impact of gastric acid suppressants on cytochrome P450 3A4 and P-glycoprotein: consequences for FK506 assimilation. Lemahieu et al., Kidney Int. 2005 Mar; 67(3):1152-60.), omerprazole (Interaction of omeprazole, lansoprazole and pantoprazole with P-glycoprotein. Pauli-Magnus et al., Naunyn Schmiedebergs Arch Pharmacol. 2001 Dec;364(6):551-7.), Vitamin E TPGS (Enhanced oral paclitaxel absorption with vitamin E-TPGS: effect on solubility and permeability in vitro, in situ and in vivo. Varma MV, Panchagnula R; Eur J Pharm Sci. 2005 Jul-Aug;25(4-5):445-53.), verapimil (Verapamil metabolites: potential P-glycoprotein-mediated multidrug resistance reversal agents. Woodland et al., Can J Physiol Pharmacol. 2003 Aug;81(8):800-5) as well as metabolites or therapeutically inactive enantiomers.

To optimise bioavailability and therapeutic benefit, in addition to combining poorly permeable drugs with permeability enhancers or solubility enhancers, there is a need to further add efflux pump inhibitors (Coadministration of Oral Cyclosporin A Enables Oral Therapy with Paclitaxel, Meerum Terwogt; Clinical Cancer Research Vol. 5, 3379-3384, November 1999). A further issue with cancer treatment is inherent immunosuppression and red blood cell death associated with several anticancer agents. Consequently, a significant side effect in anti-cancer treatment relates to immunosuppression or immunoablation wherein cells in the immune system are destroyed thereby reducing the capability of the immune system to protect the body from opportunistic infections. To prevent or overcome immunosuppression may require the co-administration of immunostimulators such as inosine or blood enriching agents such as erythropoietin. A number of approaches demonstrating the effectiveness of co-administering immunostimulatory, immunomodulatory and cytoprotecting agents with anti-cancer have been proposed and tested (Immunomodulation with interferon-gamma and colony-stimulating factors for refractory fungal infections in patients with leukemia. Dignani et al., Cancer. 2005 Jul 1;104(1):199-204; Cytoprotection and immunomodulation in cancer therapy. Diwanay et al., Curr Med Chem Anti-Canc Agents. 2004 Nov;4(6):479-90.).

Treatment of infectious diseases such as HIV/AIDS, Malaria and Tuberculosis often requires the administration of drug cocktails to effectively control the viral or bacterial titres to manageable levels. As with other cocktails, the problem associated with combinations is differential inherent drug solubility, permeability and stability profiles. Additionally, some effective drugs are susceptible to efflux pumps. Therefore, effective treatments will require the development of formats capable of bundling previously incompatible drug formulation types that improve low solubility, enhance permeability and control release of unstable drugs with short half lives. Two further issues relate to efflux and immunosuppression. Therefore, the addition of efflux inhibitors and/or immunostimulators to cocktails may improve bioavailability and the overall effectiveness of treatment.

The number of biopharmaceutical drugs in development and entering the clinic is growing at a rate double that of conventional small molecules. Most peptide and protein drugs are currently used as parenteral formulations because of their poor oral bioavailability. Development of an effective oral delivery system for these macromolecular drugs requires a thorough understanding of their physicochemical properties, such as molecular weight, hydrophobicity, ionization constants, and pH stability, as well as biological barriers that restrict protein and peptide absorption from the gastrointestinal (GI) tract, including pH variability, enzymatic degradation, and membrane efflux. Various strategies currently under investigation include amino acid backbone modifications, formulation approaches, chemical conjugation of hydrophobic or targeting ligand, and use of enzyme inhibitors, mucoadhesive polymers, and absorption enhancers. However, there is only limited success because of the hostile environment of the GI tract, including strong pH extremes and abundant presence of potent luminal enzymes (Emerging Trends in Oral Delivery of Peptide and Protein Drugs, Mahato et al., Critical Reviews™ in Therapeutic Drug Carrier Systems, Issues 2 & 3, 2003).

It has long been acknowledged that for vaccines to be effective requires the co-administration of antigenic materials with adjuvants which stimulate the immune system to identify and illicit a immunological response to the immunogenic antigen. For decades vaccines depended on adjuvants such as Alum and Freund's. A limitation with the above adjuvants is that synergies do not exist between all vaccines. Recently, a broad range of adjuvants has been developed that have demonstrated varying activity depending on the immunogenic antigen being administered. Adjuvants formulated as water in oil microemulsions are replacing the more toxic aluminum hydroxide formulations for veterinary use. These new adjuvants allow for products that are highly effective with minimal adverse reactions. One example is the development of MF59 as an adjuvant (Estuningsih SE, Smooker PM, Wiedosari E, et al. Evaluation of antigens of Fascioloa gigantica as vaccines against tropical fasciolosis in cattle. Int J Parasitol. 1997;27:1419-1428.).

A further lipid-based adjuvant class are the saponins, a heterogeneous group of sterol glycosides and triterpene glycosides which are common constituents of plants. One source of triterpenoid saponins obtained from the bark of Quillaia saponaria Molina (the soap bark tree) have been known to cause substantial enhancement of immune responses since the 1920s. Despite their use in animal vaccines, the development of saponin-based formulations for human vaccines has been impeded by their complexity and concerns about toxicity. Recently, the improved molecular understanding of the relationships between adjuvant activity, toxicity and structure of saponins and formulation of saponins into structures with reduced toxicity such as ISCOMs has led to testing in humans (ISCOMs and other saponin based adjuvants, Barr et al., Advanced Drug Delivery Reviews; Vol. 32, No. 3, pages 247-271, 1998).

A related issue pertains to oral vaccination. Despite the intestinal mucosa being rich in immune cells such as M-cells and Peyer's patches, with the exception of certain live or attenuated live vaccines such as that for Polio, oral vaccination has disappointed (Cholera toxin B subunit conjugated bile salt stabilized vesicles (bilosomes) for oral immunization. Singh et al., Int J Pharm. 2004 Jul 8;278(2):379-90; Exploiting receptor biology for oral vaccination with biodegradable particulates. Foster and Hirst, Adv Drug Deliv Rev. 2005 Jan 10;57(3):431-50; Mice fed lipid-encapsulated Mycobacterium bovis BCG are protected against aerosol challenge with Mycobacterium tuberculosis. Adwell et al., Infect Immun. 2005 Mar;73(3):1903-5). To improve the oral effectiveness will require the local release of antigens and associated adjuvants proximal to the immune cells.

Abuse of prescription drugs is a growing phenomenon. Anti-depressants, sleep-inducing agents, amphetamines and painkillers are widely abused. OxyContin, a powerful and addictive painkiller, which provides a heroin-like high, and Ritalin, a stimulant used to treat attention deficit hyperactivity disorder, are among often-misused prescription drugs. Commonly, such drugs are transformed from a pill or capsule form and injected intravenously. To counteract abuse, a number of approaches has been developed, most of which involve the addition of a noxious irritant or, alternatively, an antidote to the active ingredient with the pill or capsule excipients. When injected, the antidote inactivates the active ingredient whereas the irritant causes a burning or other unpleasant sensation at the site of action. Improving tamper proofing will require novel controlled release combination products, enabling release of the active in the small intestine for maximal absorption and release of the antidote or irritant in the colon where absorption of the active is minimal. Selected antidotes or irritants will be innocuous when released in the colon and generally will not be absorbed.

To counteract the growing antibiotic resistance problem a number of approaches is being adopted to enable delivery of the antibiotic while preventing damage to the intestinal, mainly colonic, bacterial flora. One such approach is a novel antibiotic-enzyme combination that involves co-administering an antibiotic with an enzyme which degrades and thus inactivates the antibiotic. This approach requires a delivery technology that will release the antibiotic in the upper small intestine and the enzyme at the junction of the small and large intestine.

The invention relates to various drug delivery formats that permit the development of combination therapies in a single pill format and enables once-daily administration through using various controlled release technologies and tablet formats.

A major obstacle to the development of effective and user-friendly combination pills are significant drug and formulation incompatibilities. Incompatibilities include water versus oil solubility, short versus long half-lives, food versus fasted dosing and different intestinal sites of action or absorption. This invention seeks to address these issues.

### Summary of Invention

According to the invention there is provided a pharmaceutical formulation comprising a plurality of seamless minicapsules having a diameter of from 0.5 mm to 5 mm, at least some of the minicapsules containing a methyxanthine as one active ingredient, and at least some of the minicapsules containing a corticosteriod as another active ingredient.

In one embodiment at least some of the minicapsules contain both a +methyxanthine and a corticosteroid.

The minicapsules may comprise an encapsulating medium. The encapsulating medium of at least some of the minicapsules may contain at least one of the active ingredients.

In one embodiment at least some of the minicapsules comprise a core. The core may contain at least one active ingredient.

In one embodiment at least some of the minicapsules comprise at least one layer. The layer may contain at least one active ingredient. The layer may comprise a coating to control the time and/or location of the release of the active entity.

The coated seamless minicapsules may have a diameter of from 0.5 mm to 5.0 mm, from 1.2 mm to 2.0 mm, 1.4 mm to 1.8 mm.

In one embodiment at least one coating is an immediate release coating, a sustained release coating, a sustained release and an immediate release coating, an enteric coating, or a bioadhesive coating such as a mucoadhesive coating.

In one embodiment the layer comprises a buffer layer.

In one embodiment the minicapsule is formed from a core solution containing an active ingredient, and an encapsulating solution which forms, on setting, the encapsulating medium. The encapsulating solution may contain an active ingredient. The active ingredient contained in the encapsulating solution may be the same as or different from the active ingredient in the core solution. The active ingredient contained in the encapsulating solution may be in a micronised or nanonized particle form. The minicapsule may be formed from a solution containing the encapsulating medium and an active ingredient.

In one embodiment the formulation comprises at least two different populations of minicapsules. One population of minicapsules may contain a methyxanthine and another population of minicapsules contains a corticosteroid.

In one embodiment about 50% of the formulation is comprised of a population of sustained release seamless minicapsules of a methylxanthine and a population of about 50% of a sustained release seamless minicapsule of a Corticosteroid. The ratio of seamless minicapsules of a Methylxanthine to a Corticosteroid may be about: 95:5, 90:10, 80:20, 70:30, 60:40, 50:50, 5:95, 10:90, 20:80, 30:70 or 40:60.

The invention also provides a formulation comprising a methylxanthine and a corticosteroid in a multiparticulate seamless oral formulation. The formulation may comprise sustained release particles having a core containing a methylxanthine and/or a corticosteroid in a pharmaceutically acceptable solvent or liquid phase encapsulated into multiparticulate seamless minicapsules. Alternatively the formulation may comprise sustained release particles having a core containing a methylxanthine and/or a corticosteroid in a pharmaceutically acceptable solvent or liquid phase encapsulated into multiparticulate seamless minicapsules encapsulated with a gelatine shell containing a methlyxanthine and/or a corticosteroid. The seamless minicapsules may be coated with a rate-controlling or enteric coating to achieve effective plasma levels of the methylxanthine and the corticosteroid over a period of at least 12 or 24 hours. An immediate release coating may be provided in addition to the rate-controlling coating. The immediate release coating may contain a methylxanthine. Alternatively or additionally the immediate release coating contains a corticosteroid.

In one embodiment the immediate release formulation comprises seamless minicapsules containing a core of a methylxanthine and/or a corticosteroid solubilised or suspended or dispersed in a liquid phase.

In one case the formulation comprises at least two populations of sustained release minicapsules having different in-vitro dissolution profiles. The formulation may provide a dissolution profile in a pre-determined media such that about 0-25% of the combined product is released after 1 hour, about >25% released after 3 hours, about >50% released after 6 hours and >80% released after 12 hours. Alternatively the formulation may provide a dissolution profile in a pre-determined media such that <15% of the combined product is released after 1 hour, about >15% is released after 3 hours, about >35% is released after 9 hours, about >45% is released after 12 hours and >80% is released after 24 hours.

The methylxanthine may be selected from theophylline, pentoxifylline, and A802715.

The corticosteroid may be selected from dexamethasone, prednisolone, prednisone and budesonide.

The invention also provides a capsule containing a plurality of minicapsules of the invention. The capsule may contain another entity.

In another aspect the invention provides a formulation comprising a plurality of seamless multiparticulate minicapsules having a diameter of from 0.5 mm to 5 mm, the minicapsules having an encapsulating medium, and the mincapsules containing at least two different active ingredients. The encapsulating medium of at least some of the minicapsules may contain at least one of the active ingredients. At least some of the minicapsules may comprise a core. The core may contain at least one active ingredient.

At least some of the minicapsules may comprise at least one layer. The layer may contain at least one active ingredient. The layer may comprise a coating to control the time and/or location of the release of the active entity.

The coated seamless minicapsules may have a diameter of from 0.5 mm to 5.0mum, from 1.2 mm to 3.0 mm, from 1.4 mm to 1.8 mm.

At least one coating may be an immediate release coating, a sustained release coating, a sustained release and an immediate release coating, an enteric coating, or a bioadhesive coating such as a mucoadhesive coating.

In one embodiment the layer comprises a buffer layer.

In one case the minicapsule is formed from a core solution containing an active ingredient, and an encapsulating solution which forms, on setting, the encapsulating medium. The encapsulating solution may contain an active ingredient. The active ingredient contained in the encapsulating solution may be the same as or different from the active ingredient in the core solution. The active ingredient contained in the encapsulating solution may be in a micronised or nanonized particle form.

In one embodiment at least some of the minicapsules are formed form a solution containing the encapsulating medium and an active ingredient.

The formulation may comprise at least two different populations of minicapsules. One population of minicapsules may contain a first active ingredient and another population of minicapsules may contain a second active ingredient.

The formulation may comprise a capsule containing a plurality of minicapsules. The capsule may contain another entity. The other entity may be in a liquid; powder, solid, semi-solid or gaseous form. The other entity may comprise an active entity.

In another embodiment the formulation comprises a tablet or pellet containing a plurality of minicapsules. The tablet or pellet may contain another entity. The other entity may be an active entity.

In one embodiment of the various aspects of the invention at least some of the minicapsules are provided with a bioadhesive such as a mucoadhesive.

The bioadhesive may comprise from 0% to 10% by weight of one or more of the following polymer classes:- polyacrylates; polyanhydrides; chitosans; carbopols; cellulose; methylcellulose; methylated deoxycellulose (m-doc^{™}), lectins.

The bioadhesive may comprise from 0% to 10% by weight of one or more of the following thiolated or otherwise derivated polymers:- ppolyacrylates; polyanhydrides; chitosans; carbopols; cellulose; methylcellulose; methylated deoxycellulose (m-doc^{™}), lectins.

The bioadhesive may comprise a coating. Alternatively or additioanlly the bioadhesive is incorporated into a part or layer of the minicapsule such as into the rate-controlling layer and/or into the encapsulating medium.

In another embodiment at least some of the minicapsules have have a layer such as an outer layer which is divided into a least two parts. The parts may be of the same or different compositions.

The formulation may comprise hard gelatine capsules with solid, semisolid or liquid cores.

The formulation may comprise a sachet, a sprinkle, a suppository for anal or vaginal or intrauterine delivery, a suppository or sprinkle for buccal delivery, nasal delivery or pulmonary delivery.

The present invention, seeks, through bundling in an innovative way, several approaches as novel combinations. Starting with poor solubility, adding controlled release and muco- or bio-adhesive polymers, and including more than one active or an active and an adjuvant, the current approach addresses several unmet and challenging needs.

The invention provides an oral formulation or process which can be used to administer the solubilised and/or dispersion of the active ingredient as a combination product in a manner which allows the formulation to be subsequently coated to deliver the active at a predetermined site of absorption and/or at a predetermined rate of delivery consistent with the optimum absorption and bioavailability or plasma profile of the drug.

The invention also provides an oral formulation which can be used to administer one or more active ingredient of differing solubility which, released in a predetermined manner to target sites in the gastrointestinal tract, achieve maximum absorption at the site of release of the active ingredient.

The oral formulation of the invention can provide the release of an active ingredient in the gastrointestinal tract in a manner which minimises high local concentrations of solid active ingredient, thereby reducing intestinal lining irritation. Multiparticulate drug delivery systems by their nature allow the release of the active ingredient over a larger surface area of the gastrointestinal tract thereby minimising high localised drug concentration for drugs which are irritants to the gastrointestinal tract, thus reducing any associated side effects.

This invention permits the development of broad and novel combination therapies, combining previously incompatible formulations, whether:
• water or oil soluble,
• in powder or liquid/emulsion form,
• in liquid, semi-liquid or solid form
• exhibiting different half lives,
• small molecule or biopharmaceutical,
• therapeutic or adjuvant,
• released in stomach, small or large intestine.

The minicapsule core in this invention may be comprised of a solid, a semi-solid or a liquid core. Furthermore, the minicapsules may be coated with one or several coating combinations. Coatings may contain one or more of the following:
• Active drug
• Adjuvants
• Bio- or mucoadhesive agents
• Controlled release polymers
• Taste-masking entities
• Moisture prevention or retention entities
• Oxidation prevention or retention
• Light blocking agents

To enable delivery of the complex anti-cancer drug combinations cited above requires the development of a sophisticated drug delivery aggregator or bundling technology. The present invention enables the bundling or aggregation of complex drug combinations.

The formulations of the invention permit technology bundling. The potential to formulate non-conjugated or conjugated biopharmaceuticals with or without permeability enhancing excipients, encapsulate such into minicapsules or solid minispheres and then coat with muco- or bio-adhesives and/or controlled release polymers is enabled by the current invention.

The invention provides a formulation with an antigen and an adjuvant in a minicapsule core, buffer or shell, dual coating the minicapsule with an inner mucoadhesive coat and an outer controlled release coating. The minicapsules will adhere to regions rich in M-cells and Peyer's Patches and release the adjuvant and antigen in sufficient concentrations locally to ensure a strong immune response.

The invention facilitates combinations to be developed so that prospective addicts will be unable to distinguish between capsules containing the active from those containing an antidote or irritant and thus suffer the consequences or abstain from abusing the drug.

The invention also provides a formulation for co-administration colonically of `good' bacteria with small intestinal antibiotic release.

The invention includes the following drug delivery/tablet formats:
• A bead format whereby concentric layers of drug are coated onto an inert bead
• A bead format whereby the core is comprised of an active drug that is further coated with concentric, layers of active drug
• A hard gelatine capsules containing controlled release beads
• A hard gelatine capsule containing individual drugs in mini-pellet form
• A compressed tablet wherein individual drugs are separated by means of layering
• A compressed tablet wherein individual controlled release drug beads are compressed into a tablet form

The invention permits the development of various combinations, enabling different drug release profiles, in various tablet or pill formats. The invention caters for various administrative formats such as drugs that are readily available in free-flowing powder form, and/or drugs that are optimally formulated in liquid/emulsion form, such as those exhibiting poorly soluble or poorly permeable physicochemical properties. Thus the present invention overcomes and caters for all drug formulations, including previously incompatible formulations.

Combination Therapy Advantages:
• Simper - improved compliance and treatment outcome
• Reduce error in mediacation
• Reduce resistance
• Synergistic combinations
   o Improved disease management
   o Enhanced bioavailability
   o Safety
• Reduced shortages (logistics)
• One expiry date
• Easier procurement, management and handling
• Reduced production, packaging and shipping costs
• Reduced side effects
• Improved tamper-proofing - reduced abuse

• In particular, the invention provides an oral Methylxanthine, most especially Theophylline, pentoxifylline (POF) or A802715 as a multiparticulate seamless minicapsule formulation in combination with a Corticosteroid most especially Dexamethasone, Prednisolone, Prednisone or Budesonide for twice (bid) or once daily (qd) administration to a patient, comprising sustained release particles each having a core containing Theophylline or one of the above named corticosteroids in a pharmaceutically acceptable solvent or liquid phase and encapsulated into multiparticulate seamless minicapsules in a range of 1-5 mm in diameter. The encapsulated Theophylline/Corticosteroid minicapsules are coated with a rate-controlling polymer coat-comprised of amino methacrylate copolymers in an amount sufficient to achieve therapeutically effective plasma levels of the above combined medicaments over at least 12 or 24 hours in the patient with said condition Asthma/COPD.

The product can also be formulated as an immediate release (IR) dosage form which has an Immediate Onset of Action to provide sufficient relief to the patient within a relatively short period.

The product may also be formulated to provide increased residence time in specific areas of the GIT, maximising release either in the stomach, small intestine or the colon. Controlling the release profile can have a chronotherapeutic effect, especially important in the control of nocturnal breakthrough diseases, including asthma or COPD events. Also, localised release can enhance activity of individual or combination therapies in other inflammatory conditions such as inflammatory bowel disease and Crohn's Disease.

This invention provides a product to address the issue of multiple dosing of a Methylxanthine, such as low dose (5-10 mg/L) Theophylline with a corticosteroid. The combination product should serve to increase the responsiveness of COPD, asthma and other inflammatory disease patients to a corticosteroid. The complementary actions of each drug will reduce side effects associated with the individual higher doses required for activity when either is administered alone.

The invention provides an oral combination product of a Methylxanthine, preferably either of Theophylline, pentoxifylline and A802715 and a Corticosteroid either of Dexamethasone or Prednisolone, Prednisone or Budesonide as a multiparticulate seamless minicapsule formulation for twice or once daily administration to a patient, comprising sustained release particles each having a core containing the active ingredient in a pharmaceutically acceptable solvent or liquid phase and encapsulated into seamless multiparticulate seamless minicapsules in a range of 0.50 - 5.00mm in diameter, more especially in the range 1.50 - 2.00 mm. The combination product of Methylxanthine and Corticosteroid seamless minicapsules may be coated with a rate-controlling polymer coat comprised of amino methacrylate copolymers or an Enteric Coat of a similar amino methacrylate but a different grade in an amount sufficient to achieve therapeutically effective plasma levels of the combined active drugs over at least 12 or 24 hours in the patient.

Additionally, the minicapsules may be coated with at least one or more of the following coatings:
- at least one nitrogen containing polymer, comprising of at least one polyacrylate and or/one poly-N-vinylamide and/or one poly-N-vinyl-lactame, polyacrylamide and/or polyvinylpyrrolidone being preferred
- at least one sulphur containing or thiolated polymer coating, comprising of at least one thiolated cellulose or ethylcellulose derivative and/or a thiolated polyacrylate and/or a thiolated polyacrylamide and/or a thiolated polyvinylpyrrolidone
- at least one polymer coating, comprising of at least one cellulose, ethylcellulose or cellulose analogue, chitosan or chitosan analogues being preferred

The minicapsules have a diameter in the 0.5 to 5.0 mm range, preferably in the 0.5-3:0 mm range, preferably between 1.2-2.0 mm and more preferably in the 1.4-1.8mm range

The minicapsules are capable of extended residence times in the small intestine for a period of at least 5 hours, preferably at least 7 hours and more preferably in the 8-24 hour range to enable maximal bioactivity of the core active agent, locally or systemically

The minicapsules are capable of extended residence times in the large intestine for a period of at least 5 hours, preferably at least 7 hours and more preferably in the 8-24 hour range to enable maximal bioactivity of the core active agent, locally or systemically

The minicapsules are capable of extended residence times in the gastric environment for a period of at least 5 hours, preferably at least 7 hours and more preferably in the 8-24 hour range to enable maximal bioactivity of the core active agent, locally or systemically

In one embodiment, a portion or all of the sustained release minicapsules are further coated with an immediate release a Methylxanthine, one of theophylline, pentoxifylline or A802715 or a Corticosteroid, one of dexamethasone, prednisolone, predisone or Budesonide solution onto the rate-controlling polymer coat.

In an alternative embodiment, the formulation can contain a portion of immediate release seamless minicapsules each comprising a core of a methylxanthine, e.g. theophylline, pentoxifylline or A802715 or a Corticosteroid either of the following dexamethasone, prednisolone, prednisone or Budesonide solubilised or suspended or dispersed in a liquid phase.

In one embodiment the formulation comprises at least two populations of sustained release minicapsules having different in-vitro dissolution profiles.

In one embodiment the formulation provides a dissolution profile in a pre-determined media such that about 0-25% of the combined product is released after 1 hour, about >25% released after 3 hours, about >50% released after 6 hours and >80% released after 12 hours.

In an alternative embodiment, the formulation provides a dissolution profile in a pre-determined media such that <15% of the combined product is released after 1 hour, about >15% is released after 3 hours, about >35% is released after 9 hours, about >45% is released after 12 hours and >80% is released after 24 hours.

In one case, greater than 80% of the formulation is comprised of sustained release minicapsules.

In a preferred embodiment 50% of the formulation is comprised of a population of sustained release seamless minicapsules of a methylxanthine of either theophylline, pentoxifylline or A802715 and a population of 50% of a sustained release seamless minicapsule of a Corticosteroid, in this case either Dexamethasone or Prednisolone or Prednisone or Budesonide.

In a preferred embodiment, the ratio of seamless minicapsules of a Methylxanthine to a Corticosteroid can be formulated according to the following formulae: - 95:5/90:10/80:20/70:30/60:40/50:50/5:95/10:90/20:80/30:70/40:60

The rate-controlling polymer coat may contain Ammonio Methacrylate Copolymer as described in USP /NF in the following ratio's 5:95; 10:90;15:85 as a mixture of Eudragit RL:Eudragit RS more especially Eudragit RL 12.5:Eudragit RS 12.5

The Enteric Coating Polymer may be Eudragit S 12.5 or Eudragit S 30D

The rate-controlling polymer coat may be of Eudragit S 12.5 providing 0 drug release in the stomach for up to 2-6 hours.

The invention also provides a formulation in which a percentage of the enteric coated Corticosteroid seamless minicapsules (Example 3) and a percentage of the coated Methylxanthine seamless minicapsules (Example 2) were blended as per in Example 1.The said blended components were filled into suitable hard gelatin capsules to the required target dosage strength.

In one embodiment the Methylxanthine seamless minicapsules, of either theophylline, pentoxifylline or A802715 as per Example 2 is filled into hard gelatin capsules as a single component to the required target dosage for the products specified indication, excluding the Corticosteroid component.

In another embodiment the Corticosteroid seamless minicapsules in this case either Dexamethasone or Prednisolone or Prednisone or Budesonide as per Example 3 is filled into hard gelatin capsules as a single component to the required target dosage for the products specified indication, excluding the Methylxanthine of either theophylline, pentoxifylline or A802715

In one case, the combination minicapsule formulations may have an increased residence time and release the actives locally in the small intestine

The combination minicapsule formulations may have an increased residence time and release the actives locally in the colon

The combination minicapsule formulations may have an increased residence time and release the actives locally in the stomach

The coatings may be modulated to permit time specific release, thereby enabling chronotherapies for circadian related diseases, especially nocturnal breakout disease conditions and cardiovascular conditions.

In one embodiment a methylxanthine is combined with other immunostimulatory or immunomodulatory actives, including for example cyclosporine.

In other embodiments a methylxanthine is combined with either of one or a combination of the following actives, including anticancer agents, proapoptotic agents, Vitamin A analogues, Nitric Oxide Donors or Reactive Oxygen Species Scavengers.

More generally, the invention provides an oral multiple minicapsule-based product, each minicapsule comprising at least one layer, the core of each minicapsule comprising water-soluble, water-insoluble or partially water-soluble actives in liquid or solid form, a buffer layer comprise of a suitable oil, coated with one or several coats comprised rate-releasing, mucoadhesive or bioadhesive coatings alone or blended, in any combination thereof.

The liquid may be selected from the group consisting of a solution, a spirit, an elixir, a spray, a syrup, an emulsion, a microemulsion, a nanoemulsion, a nanosuspension, a wax, an aerosol, a gel, a foam, a solid foam and a fluid extract.

The shell or solid core may be formed of a material selected from the group consisting of gelatin, starch, casein, chitosan, soya bean protein, safflower protein, alginates, gellan gum, carrageenan, xanthan gum, phtalated gelatin, succinated gelatin, cellulosephtalate-acetate, oleoresin, polyvinylacetate, hydroxypropyl methyl cellulose, polymerisates of acrylic or methacrylic esters, polyvinylacetate-phtalate and combinations thereof.

The minicapsules may be coated with at least one coating comprising at least one nitrogen-containing polymer and/or sulphur-containing or thiolated polymer and/or cellulose or cellulose polymer coating.

The nitrogen-containing coating may be selected from one or more of:
a polyacrylate; a poly-N-vinylamide; and a poly-N-vinyl-lactame.

The nitrogen-containing polymer may be a polyacrylamide or polyvinylpyrollidone.

The cellulose or cellulose derived polymer may be selected from any one or more of
an ethylcellulose or derivative
a methylcellulose or derivative
a chitosan or derivative.

The mucoadhesive or bioadhesive is selected from any one or more of: Polyacrylates; Polyanhydrides; Lectins; Chitosan; Chitosan glutamate; Polycarbophil; and Carbopol™

The sulphur-containing polymer may be selected from any one or more of: a thiolated cellulose or ethylcellulose derivative; a thiolated polyacrylate; a thiolated polyacylamide; and a thiolated polyvinylpyrollidone.

The polymer coating may be one or more controlled release, mucoadhesive or bioadhesive polymer in any combination or blend.

The multiparticulate capsules may include an antimicrobial selected from the group consisting of paraben and sorbic acid.

The ingredient introduced in said primary capsule comprises a moisture content in the range of about 0% to 6% by weight, generally about 0% to 3% by weight.

Primary and secondary capsules may contain at least one pharmaceutically acceptable lubricant in the range of about 0% to 10% by weight. The lubricant may be selected from the group consisting of aluminiumstearate, calciumstearate, magnesiumstearate, tinstearate, talc, sodium lauryl sulfate, lecithins, mineral oils, stearic acid, silicones and combinations thereof.

In one embodiments the coatings are modulated to permit time specific release, thereby enabling chronotherapies for circadian related diseases, including, but not limited to, nocturnal breakout disease, cardiovascular disease, asthma, respiratory conditions, CNS, autoimmune diseases such as rheumatoid arthritis and oesteoarthritis.

The coatings may be modulated to permit in addition to specific time release modified transit times in the oesophagus, gastric, small intestine, colon or rectum, through the inclusion of muco- or bio-adhesives in the gelatine shell or coated, alone or in combination with controlled release polymers, onto the gelatine shell.

The formulations may comprise another active ingredient such as other immunostimulatory or immunomodulatory actives, including for example cyclosporine, tacrolimus, sacrolimus, inosine or derivates thereof.

The formulation may be encapsulated into hard gelatin capsules, filled into a sachet, used as a sprinkle or as a suppository.

These and other features are included in the claims which are incorporated therein.

### Detailed Description

The invention provides oral Methylxanthines, most especially in this case Theophylline, pentoxifylline or A802715 multiparticulate seamless minicapsule formulation in combination with corticosteroids, most especially Dexamethasone, Prednisolone, Prednisone or Budesonide for twice or once daily administration to a patient. The product comprises sustained release particles each having a core containing one or both of the active ingredients in a solvent or liquid phase as a seamless minicapsule, the core being coated with a rate-controlling polymer coat comprised of ammonia methacrylate copolymers in an amount sufficient to achieve therapeutically effective plasma levels of the combination product over at least 12 or 24 hours. In another embodiment, the core may contain an active ingredient, for example a corticosteroids, in a solvent or liquid phase with another drug, for example a methylxanthine embedded in the gelatine shell, blended with the various shell coatings or included with the intermediate buffering oil layer. Additionally, the coating may contain mucoadhesives which increase the residence time at particular GIT locations. Such mucoadhesives may be applied together with rate-releasing polymers or added as an individual coat, over or under the rate-releasing coat.

In a further embodiment, the core is comprised of a solid, formed from molten gelatine or gelatine-like seamless spherical particles, these particles being coated with rate-limiting polymer coatings or muco-/bio-adhesives in any blend or number of alternating coatings. Yet another embodiment is composed of a solid or semisolid (waxy) material that is liquid at process temperature but solid or semi-solid at room temperature, these particles being coated with rate-limiting polymer coatings or muco-/bio-adhesives in any blend or number of alternating coatings.

The Methylxanthine and the Corticosteroid seamless minicapsules were manufactured according to Freund Industrial Co, Ltd. US Patent No 5,882,680 (Seamless Capsule and Method of Manufacturing the Same), the entire contents of which are incorporated herein by reference.

The principle of seamless minicapsule formation is the utilisation of "surface tension", when two different solutions (which are not or hardly dissolved with each other) contact each other, which works by reducing the contact area of the two different solutions.

After encapsulating the core solution which is ejected through an orifice with a certain diameter, with the shell solution which is also ejected through an outer orifice, the encapsulated sphere is then ejected into a cooling or hardening solution and the outer shell solution is gelled or solidified. This brief describes the formation of seamless minicapsules.

The core solution is mainly a hydrophobic solution or suspension. The outer shell solution is normally gelatin based. However a hydrophilic solution can also be encapsulated with the existence of an intermediate solution, which can avoid the direct contact of the hydrophilic core solution with the outer shell.

With the nozzle having a single orifice, a minicapsule or a bead of shell/core mixed suspension can be processed.

With the nozzle having two orifices (centre and outer),a hydrophobic solution can be encapsulated.

With the nozzle having three or more orifices seamless minicapsules for various applications can be processed. (Ref US Patent No.5,882,680)

By using the above described manufacturing processing method as per US patent No.5,882,680 for multiparticulate seamless minicapsules, nifidipine multiparticulate seamless minicapsules were produced. The completed seamless minicapsules preferably have an average diameter of 0.50 - 5.00mm,more especially in the range 1.50 -1.80mm.

According to one embodiment a portion or all of the sustained release particles further comprise an immediate release coating applied onto the rate-controlling polymer coat, which immediate release coating comprises solubilised Methylxanthine or Corticosteroid in a liquid phase.

In an alternative embodiment, the formulation can contain a portion of immediate release minicapsules each comprising a core of a Methylxanthine or a Corticosteroid solubilised in a liquid phase.

The formulation according to the invention may also comprise at least two populations of sustained release seamless minicapsules having two different in vitro dissolution profiles.

Also preferably, the formulation according to the invention provides a dissolution profile in a pre-selected media such that about 0-25% of the combined product is released after 1 hour; about >25% after 3 hours; about >50% after 6 hours; >80 after 12 hours.

In an alternative embodiment the formulation provides a dissolution profile in a pre-determined media such that about <15% of the combined product is released after 1 hour; about >15% is released after 3 hours;> 35% is release after 9 hours; about >45% is released after 12 hours and at least 80% is released after 24 hours.

In a preferred embodiment greater than 80% of the formulation is comprised of sustained release seamless minicapsules.

In a preferred embodiment 50% of the formulation is comprised of a population of sustained release seamless minicapsules of a Methylxanthine, in this case Theophylline, pentoxifylline or A802715 and a population of 50% of a sustained release seamless minicapsule of a Corticosteroid, in this case either Dexamethasone, Prednisolone, Prednisone or Budesonide.

In a preferred embodiment the ratio of seamless minicapsules of a Methylxanthine: Corticosteriod can be formulated in the following ratio's:- 95:5; 90:10; 80:20;70:30; 60:40;50:50 and so on.

In a preferred embodiment the rate-controlling polymer coat contains Ammonia Methacrylate Copolymer Type A and Ammonia Methacrylate Copolymer Type B as described in USP/NF.

Such copolymers are manufactured and marketed by Rohm, GmbH, Darmstadt, Germany.

Most preferably the rate-controlling polymer coat contains a 5:95 or 10:90 or 15:85 mixture ofEudragit RL:Eudragit RS most especially Eudragit RL 12.5:Eudragit RS 12.5 or Eudragit RL30D:Eudragit RS30D or Eudragit E100 or Eudragit E PO or a combination thereof.

Preferably the sustained release seamless minicapsules following application of the rate-controlling polymer coat are dried at a temperature of about 35-55 deg C for between 12-24 hours

In a preferred embodiment the formulation is encapsulated, for example in a hard gelatin capsule.

The sustained release seamless minicapsules are formed by coating the active seamless minicapsule with the rate-controlling polymer coat comprised of ammonio methacrylate copolymers such as those sold under the Trade Mark EUDRAGIT.

EUDRAGIT polymers are polymeric lacquer substances based on acrylates and/or methacrylates. The polymeric materials sold under the Trade Mark EUDRAGIT RL and EUDRAGIT RS are acrylic resins comprising copolymers of acrylic and methacrylic acid esthers with a low content of quaternary ammonium groups and are described in the "EUDRAGIT" brochure of Degussa GmbH wherein detailed physical-chemical data of these products are given. The ammonium groups are present as salts and give rise to the permeability of the lacquer films. EUDRAGIT RL is freely permeable or RS slightly permeable, independent of pH.

Minicapsules with both pH- and time-controlled release (double coating with EUDRADIT® RL/RS and EUDRAGIT® FS30D) may be prepared according to Degussa (In-vivo evaluation of EUDRAGIT™ - A novel pH- and time-controlled multiple unit colonic drug delivery system, Skalsky B., et al, Controlled Release Society 31^{st} Annual Meeting TRANSACTIONS).

The mucoadhesive controlled GIT transit minicapsules are formed by coating the active seamless minicapsule with the transit-controlling polymer coat comprised of, for example various cellulose or cellulose derivatives such as chitosan or those sold under the brand name Carbapol®.

The rate-controlling polymer coat maybe built up by applying a plurality of coats of polymer solution or suspension to the minicapsule as hereafter described. The polymer solution or suspension contains the polymer(s) dissolved or suspended, respectively in a suitable aqueous or organic solvent or mixture of solvents, optionally in the presence of a lubricant. Suitable lubricants are talc, stearic acid, magnesium stearate and sodium stearate. A particularly preferred lubricant is talc.

The polymer solution or suspension may optionally include a plasticizing agent. Suitable plasticizing agents include polyethylene glycol, propyleneglycol, glycerol, triacetin, dimethyl phthalate, diethyl phthalate, dibutyl phthalate, dibutyl sebacate or varying percentages of acetylated monoglycerides.

Suitable organic solvents include isopropyl alcohol (IPA) or acetone or a mixture.

The polymer solution or suspension maybe applied to the minicapsules preferably using an automated system such as a GLATT fluidised bed processor, Vector Flow Coater System or an Aeromatic or a Vector Granurex Rotor Processor System.

Polymer solution/suspension in the quantity of 5-75 ml per kilogram of minicapsules maybe applied to the minicapsules using one of the listed automated fluidised bed processing systems to given target polymer coating weight.

In accordance with the invention the drug loaded minicapsules are coated with the rate-controlling polymers to achieve a target dissolution rate. The drug released from these minicapsules is diffusion controlled as the polymer swells and becomes permeable, it allows for the controlled release in the GIT. In order to achieve a suitable dissolution profile, the following parameters require consideration, efficient process/conditions, drug solubility/particle size, minicapsule surface area, minicapsule diameter and coating polymer suitability.

### Example 1

| Core Solution | |
|---|---|
| -- Corticosteroid(Dexamethasone, Prednisolone, Prednisone or Budesonide) | 50-200 grams |
| --PEG 400 | 50-500 grams |
| -- Ethanol | 0-500 grams |
| --Vegetable or Mineral Oil | 1000 grams |

| Film Solution | |
|---|---|
| -- Methylxanthine (e.g. Theophylline) | 30-50 %/wt |
| -- Gelatin | 18 %/wt |
| -- Sorbitol | 2 %/wt |
| -- Purified Water | as required |

| Polymer Coating Solution | |
|---|---|
| -- Eudragit RL | 5% w/w |
| -- Eudragit RS | 95% w/w |
| -- Talc | as required |
| -- Minicapsule diameter | 1.50 - 2.00mm |

The Methylxanthine and the Corticosteroid Combination Multiparticulate Seamless Minicapsules were manufactured according to Freund Industrial Co. Ltd. US Patent No. 5,882,680 (Seamless Capsule and Method of Manufacturing Same) and as described in the Summary of the Invention Section.

In order to coat the core seamless minicapsules, a coating solution of 6.25% Eudragit RL (5% w/w) and Eudragit RS (95% w/w) dissolved in isopropyl alcohol/acetone mixture was sprayed onto the minicapsules using an automated fluidised bed processor. Talc was added simultaneously to avoid agglomeration.

The coated minicapsules were dried in an environmentally controlled drier at 35-55 deg C for between 12-24 hours to remove any residual solvents and enhance the functionality of the Eudragit coating.

Encapsulation 10-90% immediate release: 10-90% sustained release, most preferably a 50:50 ratio.

Methylxanthine/Corticosteroid seamless minicapsules uncoated (50% w/w by potency) and the polymer coated minicapsules (50% w/w by potency) from the above were blended using a suitable mechanical blender.

The resultant blend was filled into suitable gelatin capsules to the required target strength.

### Example 2

| Core Solution | |
|---|---|
| -- Methlyxanthine (Theophylline, pentoxifylline or A802715 USP/EP) | 800 grams |
| -- Gelatin | 1100 grams |
| -- Sorbitol | 100 grams |
| -- Purified Water | 4200 grams |

| Polymer Coating solution | |
|---|---|
| -- Eudragit RS | 95% w/w |
| -- Eudragit RL | 5% w/w |
| -- Diethylphthalate | 5-10% w/w |
| -- Talc | as required |
| -- Minicapsule Diameter | 1.50 - 2.00mm |

The above seamless minicapsules were manufactured in the same way as Example 1 with the following exceptions:-
1. The Methylxanthine was added into the core solution and was treated with a High Pressure Homogeniser.
   The median and film solutions were excluded from this example.
2. The polymer solution included a 5-10% plasticiser.

### Example 3

| Core Solution | |
|---|---|
| -- Corticosteroid (Dexamethasone, Prednisolone, Prednisone or Budesonide) | 5-50 %/wt |
| -- PEG (200;300;400;600) | 50-95 %/wt |
| -- MCT (Medium Chain Fatty Acid Trigliceride) | 100 %/wt |

| Film Solution | |
|---|---|
| -- Gelatin | 10 - 25 %/wt |
| -- Sorbitol | 1 -5 %/wt |
| -- Purified Water | 50 -100 %/wt |

| Polymer Coating Solution | |
|---|---|
| -- Eudragit S | 100 %/wt |
| -- Isopropyl Alcohol/acetone | as required |
| -- Talc | as required |
| -- Minicapsule Diameter | 1.50 - 2.00mm |

The above seamless minicapsules were manufactured in the same way as Example 1 with the following exceptions:-
1. The core solution was pre-treated with an Ultra Centrifugal Mill.
2. Eudragit S was used as the polymer coat to provide an enteric coat with 0 drug release of up to 2-6 hours to the minicapsules, to target the drug release to the GIT and providing a pulsed release profile.

A percentage of the Enteric Coated Corticosteroid (Example 3) seamless minicapsules and a percentage of the coated Methylxanthine seamless minicapsules from Example 2 were blended as per in Example 1 and filled into suitable gelatin capsules to the target strength.

### Example 3a

Core and film is as per Example 3.

| Mucoadhesive Coating Solution | |
|---|---|
| -- Ethylcellulose | 5-20g |
| -- PVP | 0.5-5g |
| -- Castor Oi1 | 0-5g |
| -- Magnesium Stearate | 0.5-3 g |
| -- Aceton | 50-300g |
| -- Isopropanol | 5-50g |

| Polymer Coating Solution | |
|---|---|
| -- Eudragit RL | 5% w/w |
| -- Eudragit RS | 95% w/w |
| -- Minicapsule diameter | 1.50mm |

The Corticosteroid Multiparticulate Seamless Minicapsules were manufactured according to Freund Industrial Co. Ltd US Patent No. 5,882,680 (Seamless Capsule and Method of Manufacturing Same) and as described in the Summary of the Invention Section.

To apply a mucoadhesive coating, a coating solution of 7.0% ethylcellulose, 0.85% PVP and 1.0% Magnesium Stearate was dissolved in an isopropanil/acetone mixture was sprayed onto the minicapsules using an automated fluidised bed processor. Anti-agglomeration powder was applied to prevent agglomeration of the minicapsules, The coated minicapsules were dried in an environmentally controlled drier for between 12 to 24 hours to remove any residual solvents

In order to further coat the mucoadhesive coated seamless minicapsules, a coating solution of 6.25% Eudragit RL (5% w/w) and Eudragit RS (95% w/w) dissolved in isopropyl alcohol/acetone mixture was sprayed onto the minicapsules using an automated fluidised bed processor. Talc was added simultaneously to avoid agglomeration.

The coated minicapsules were dried in an environmentally controlled drier for between 12 to 24 hours to remove any residual solvents

Encapsulation 0-100% immediate release/100-0% sustained release.

Corticosteroid seamless minicapsules uncoated (10% w/w by potency) and the polymers coated minicapsules (90% w/w by potency) from the above were blended using a suitable mechanical blender as per Example 3. The resultant blend was filled into suitable gelatin capsules or sprinkle format to the required target strength.

### Example 4

| Core Solution | |
|---|---|
| -- Corticosteroid (Dexamethasone, Prednisolone or Prednisone) | 50-200 grams |
| -- peg 400 | 50-500 grams |
| -- Ethanol | 0-500 grams |
| --Vegetable or Mineral Oil | 1000 grams |

| Film Solution | |
|---|---|
| -- Methylxanthine (e.g. Theophylline) | 30-50 %/wt |
| -- Gelatin | 18 %/wt |
| -- Sorbitol | 2 %/wit |
| -- Purified Water | as required |

| Median Solution | |
|---|---|
| --Vegetable or Mineral Oil | 1000 grams |

| Film Solution | |
|---|---|
| -- Gelatin | 225 grams |
| -- Sorbitol | 25 grams |
| -- Purified Water | 750 grams |

| Mucoadhesive Coating Solution | |
|---|---|
| -- Ethylcellulose | 5-20g |
| -- PVP | 0.5-5g |
| -- Castor Oil | 0-5g |
| -- Magnesium Stearate | 0.5-3 g |
| -- Aceton | 50-300g |
| -- Isopropanol | 5-50g |

| Rate-Release Polymer Coating Solution | |
|---|---|
| -- Eudragit RL | 5% w/w |
| -- Eudragit RS | 95% w/w |
| -- Minicapsule diameter | 1.50mm |

The Methylxantine and the Corticosteroid Combination Multiparticulate Seamless Minicapsules were manufactured according to Freund Industrial Co. Ltd. US Patent No. 5,882,680 (Seamless Capsule and Method of Manufacturing Same) and as described in the Summary of the Invention Section.

In order to coat the core seamless minicapsules, a coating solution of 6.25% Eudragit RL (5% w/w) and Eudragit RS (95% w/w) dissolved in isopropyl alcohol/acetone mixture was sprayed onto the minicapsules using an automated fluidised bed processor. Talc was added simultaneously to avoid agglomeration.

To apply a mucoadhesive coating, a coating solution of 7.0% ethylcellulose, 0.85% PVP and 1.0% Magnesium Stearate was dissolved in an isopropanil/acetone mixture was sprayed onto the minicapsules using an automated fluidised bed processor. Anti-agglomeration powder was applied to prevent agglomeration of the minicapsules. The coated minicapsules were dried in an environmentally controlled drier for between 12 to 24 hours to remove any residual solvents

In order to further coat the mucoadhesive coated seamless minicapsules, a coating solution of 6.25% Eudragit RL (5% w/w) and Eudragit RS (95% w/w) dissolved in isopropyl alcohol/acetone mixture was sprayed onto the minicapsules using an automated fluidised bed processor. Talc was added simultaneously to avoid agglomeration.

The coated minicapsules were dried in an environmentally controlled drier at 35-55 deg C for between 12-24 hours to remove any residual solvents and enhance the functionality of the Eudragit coating.

Encapsulation 10-90% immediate release: 10-90% sustained release, most preferably a 50:50 ratio.

Methylxanthine/Corticosteroid seamless minicapsules uncoated (50% w/w by potency) and the polymer coated minicapsules (50% w/w by potency) from the above were blended using a suitable mechanical blender.

The resultant blend was filled into suitable gelatin capsules to the required target strength.

## Claims

1. A pharmaceutical formulation comprising a plurality of seamless minicapsules having a diameter of tron, 0.5 mm to 5 mm, at least some of the minicapsules containing a methyxanthine as one active ingredient, and at least some of the minicapsules containing a corticosteriod as another active ingredient.

2. A formulation as claimed in claim 1 wherein at least some of the minicapsules contain both a methyxanthine and a corticosteroid.

3. A formulation as claimed in claim 1 or 2 wherein the minicapsules comprise an encapsulating medium, in, one case the encapsulating medium of at least some of the minicapsules contains at least one of the active ingredients.

4. A formulation as claimed in any of claims 1 to 3 wherein at least some of she minicapsules comprise a core, in one case the core contains at least one active ingredient.

5. A formulation as claimed in any of claims 1 to 4 wherein at least some of the minicapsules comprise at least one layer, the layer may contain at least one active ingredient.

6. A formulation as claimed in claim 5 wherein the layer comprises a coating to control the time and/or location of the release of the active entity, in one case the coated seamless minicapsules have a diameter of from 0.5 mm to 5.0 mm, in one case the coated seamless minicapsules have a diameter of from 1.2 mm to 2.0 mm, in one case the coated seamless minicapsules have a diameter of from 1.4 mm to 1.8 mm.

7. A formulation as claimed in claim 6 wherein at least one coating is an immediate release coating, at least one coating may be a sustained release coating, in one case the coating comprises a sustained release and an immediate release coating, in one case at least one coating is an enteric coating.

8. A formulation as claimed in any of claims 1 to 7 wherein at least one coating is a bioadhesive coating such as a mucoadhesive coating.

9. A formulation as claimed in any of claims 5 to 8 wherein the layer comprises a buffer layer.

10. A formulation as claimed in any of claims 3 to 9 wherein the minicapsule is formed from a core solution containing an active ingredient, and an encapsulating solution which forms, on setting, the encapsulating medium, in one case the encapsulating solution contains an active ingredient, in one case the active ingredient contained in the encapsulating solution is the same as the active ingredient in the core solution, in one case the active ingredient contained in the encapsulating solution is different from the active ingredient in the core solution, in one case the active ingredient contained in the encapsulating solution is in a micronised or nanonized particle form.

11. A formulation as claimed in any of claims 3 to 10 wherein the minicapsule is formed form a solution containing the encapsulating medium and an active ingredient.

12. A formulation as claimed in any of claims 1 to 1 comprising at least two different populations of minicapsule, in one case one population of minicapsules contains a methyxanthine and another population of minicapsules contains a corticosteroid, in one case about 50% of the product is comprised of a population of sustained release seamless minicapsules of a methylxanthine and a population of about 50% of a sustained release seamless minicapsule of a Corticosteroid, in one case the ratio of seamless minicapsules of a Methylxanthine to a Corticosteroid is about: 95:5, 90:10, 80:20, 70:30, 60:40, 50:50, 5:95, 10:90, 20:80, 30:70 or 40:60.

13. A formulation comprising a methylxanthine and a corticosteroid in a multiparticulate seamless oral formulation.

14. A formulation as claimed in claim 13 comprising sustained release particles having a core containing a methylxanthine and/or a corticosteroid in a pharmaceutically acceptable solvent or liquid phase encapsulated into multiparticulate seamless minicapsules.

15. A formulation as claimed in claim 13 comprising sustained release particles having a core containing a methylxanthine and/or a corticosteroid in a pharmaceutically acceptable solvent or liquid phase encapsulated into multiparticulate seamless minicapsules encapsulated with a gelatine shell containing a methlyxanthine and/or a corticosteroid.

16. A formulation as claimed in any of claims 13 to 15 wherein the seamless minicapsules arc coated with a rate-controlling or enteric coating to achieve effective plasma levels of the methylxanthine and the corticosteroid over a period of at least 12 or 24 hours, in one case an immediate release coating is provided in addition to the rate-controlling coating, in one case the immediate release coating contains a methylxanthine, in one case the immediate release coating contains a corticosteroid, in one case the immediate release formulation comprises seamless minicapsules containing a core of a methylxanthine and/or a corticosteroid solubilised or suspended or dispersed in a liquid phase.

17. A formulation as claimed in any of claims 1 to 16 which comprises at least two populations of sustained release minicapsules having different in-vitro dissolution profiles, in one case the formulation provides a dissolution profile in a pre-determined media such that about 0-25% of the combined product is released after 1 hour, about >25% released after 3 hours, about >50% released after 6 hours and >80% released after 12 hours, in another cast the formulation provides a dissolution profile in a pre-determined media such that <15% of the combined product is released after 1 hour, about >15% is released after 3 hours, about >35% is released after 9 hours, about >45% is released after 12 hours and >80% is released after 24 hours.

18. A formulation as claimed in any of claims 1 to 17 wherein the methylxanthine is selected from theophylline, pentoxifylline, and A802715.

19. A formulation as claimed in any of claims 1 to 18 wherein the corticosteroid is selected from dexamethasone, prednisolone, prednisone and budesonide.

20. A formulation as claimed in any of claims 1 to 19 comprising a capsule containing a plurality of minicapsules.

21. A formulation as claimed in claim 20 wherein the capsule contains another entity.

22. A formulation as claimed in any of claims 1 to 19 comprising a capsule containing a plurality of minicapsules, the capsule may contain another entity, the other entity may be in a liquid, powder, semi-solid, solid or gaseous form, the other entity may comprise an active entity.

23. A formulation as claimed in any of claims 1 to 17 wherein at least some of the minicapsules are provided with a bioadhesive such as a mucoadhesive, in one case the bioadhesive comprises from 0% to 10% by weight of one or more of the following polymer casses:- polyacrylate; polyanhydrides; chitosans; carbopols; cellulose; methylcellulose; methylated deoxycellulose (m-doc™⁾, lectins, in another case the bioadhesive comprises from 0% to 10% by weight of one or more of the following thiolated or otherwise derivated polymers:- ppolyacrylates; polyanhydrides; chitosans, carbopols; cellulose; methylcellulose; methylated deoxycellulose (m-doc™⁾, lectins, in one case the bioadhesive comprises a coating.

24. A formulation as claimed in claim 23 wherein the bioadhesive is incorporated into a part or layer of the minicapsule, in one case the bioadhesive is incorporated into a rate-controlling layer, in another case the bioadhesive is incorporated into the encapsulating medium.

25. A formulation as claimed in any of claims 1 to 24 wherein at least some of the minicapsules have a layer such as an outer layer which is divided into a least two parts, in one case the parts are of the same composition, in one case at least some of the parts have different composition.

26. A formulation as claimed in any of claims 1 to 25 comprising hard gelatine capsules with solid, semisolid or liquid cores.

27. A formulation as claimed in any of claims 1 to 26 comprising a sachet, or a sprinkle, or a suppository for anal or vaginal delivery or intrauterine delivery, or a suppository or sprinkle for buccal delivery, nasal delivery or pulmonary delivery.

## Patentansprüche

1. Pharmazeutische Formulierung, die eine Vielzahl von nahtlosen Minikapseln mit einem Durchmesser von 0,5 mm bis 5 mm umfasst, wobei mindestens einige der Minikapseln Methylxanthin als einen Wirkstoff enthalten und mindestens einige der Minikapseln ein Corticosteroid als einen weiteren Wirkstoff enthalten.

2. Formulierung nach Anspruch 1, worin mindestens einige der Minikapseln sowohl ein Methylxanthin als auch ein Corticosteroid enthalten.

3. Formulierung nach Anspruch 1 oder 2, worin die Minikapseln ein verkapselndes Medium umfassen, in einem Fall das verkapselnde Medium von mindestens einigen Minikapseln mindestens einen der Wirkstoffe enthält.

4. Formulierung nach einem der Ansprüche 1 bis 3, worin mindestens einige der Minikapseln einen Kern umfassen, in einem Fall der Kern mindestens einen Wirkstoff enthält.

5. Formulierung nach einem der Ansprüche 1 bis 4, worin mindestens einige der Minikapseln mindestens eine Schicht umfassen, die Schicht mindestens einen Wirkstoff enthalten kann.

6. Formulierung nach Anspruch 5, worin die Schicht einen Überzug zur Kontrolle der Zeit und/oder des Orts der Freisetzung der Wirkeinheit umfasst, in einem Fall die überzogenen nahtlosen Minikapseln einen Durchmesser von 0,5 mm bis 5,0 mm haben, in einem Fall die überzogenen nahtlosen Minikapseln einen Durchmesser von 1,2 mm bis 2,0 mm haben, in einem Fall die überzogenen nahtlosen Minikapseln einen Durchmesser von 1,4 mm bis 1,8 mm haben.

7. Formulierung nach Anspruch 6, worin mindestens ein Überzug ein sofort freisetzender Überzug ist, mindestens ein Überzug ein retardiert freisetzender Überzug sein kann, in einem Fall der Überzug einen retardiert freisetzenden und einen sofort freisetzenden Überzug umfasst, in einem Fall mindestens ein Überzug ein magensaftresistenter Überzug ist.

8. Formulierung nach einem der Ansprüche 1 bis 7, worin mindestens ein Überzug ein bioadhäsiver Überzug, wie zum Beispiel ein mukoadhäsiver Überzug, ist.

9. Formulierung nach einem der Ansprüche 5 bis 8, worin die Schicht eine Pufferschicht umfasst.

10. Formulierung nach einem der Ansprüche 3 bis 9, worin die Minikapsel aus einer Kernlösung, die einen Wirkstoff enthält, und einer verkapselnden Lösung, die beim Setzen das verkapselnde Medium bildet, gebildet wird, in einem Fall die verkapselnde Lösung einen Wirkstoff enthält, in einem Fall der in der verkapselnden Lösung enthaltene Wirkstoff der gleiche wie der Wirkstoff in der Kernlösung ist, in einem Fall der in der verkapselnden Lösung enthaltene Wirkstoff verschieden von dem Wirkstoff in der Kernlösung ist, in einem Fall der in der verkapselnden Lösung enthaltene Wirkstoff in einer mikronisierten oder nanonisierten Partikelform vorliegt.

11. Formulierung nach einem der Ansprüche 3 bis 10, worin die Minikapsel aus einer Lösung gebildet wird, die das verkapselnde Medium und einen Wirkstoff enthält.

12. Formulierung nach einem der Ansprüche 1 bis 11, die mindestens zwei verschiedene Gesamtheiten von Minikapseln umfasst, wobei in einem Fall eine Gesamtheit von Minikapseln ein Methylxanthin enthält und eine weitere Gesamtheit von Minikapseln ein Corticosteroid enthält, in einem Fall etwa 50 % des Produkts aus einer Gesamtheit von retardiert freisetzenden, nahtlosen Minikapseln eines Methylxanthins und einer Gesamtheit von etwa 50 % aus einer retardiert freisetzenden, nahtlosen Minikapsel eines Corticosteroids besteht, in einem Fall das Verhältnis von nahtlosen Minikapseln von einem Methylxanthin zu einem Corticosteroid etwa Folgendes beträgt: 95 : 5, 90 : 10, 80 : 20, 70 : 30, 60 : 40, 50 : 50, 5 : 95, 10 : 90, 20 : 80, 30 : 70 oder 40 : 60.

13. Formulierung, die ein Methylxanthin und ein Corticosteroid in einer multipartikulären, nahtlosen, oralen Formulierung umfasst.

14. Formulierung nach Anspruch 13, die retardiert freisetzende Partikel umfasst, die einen Kern besitzen, der ein Methylxanthin und/oder ein Corticosteroid in einem pharmazeutisch verträglichen Lösungsmittel oder einer pharmazeutisch verträglichen, flüssigen Phase enthält, die zu multipartikulären, nahtlosen Minikapseln verkapselt sind.

15. Formulierung nach Anspruch 13, die retardiert freisetzende Partikel umfasst, die einen Kern besitzen, der ein Methylxanthin und/oder ein Corticosteroid in einem pharmazeutisch verträglichen Lösungsmittel oder einer pharmazeutisch verträglichen, flüssigen Phase enthält, die zu multipartikulären, nahtlosen Minikapseln verkapselt sind, die mit einer Gelatinehülle, die ein Methylxanthin und/oder ein Corticosteroid enthält, verkapselt sind.

16. Formulierung nach einem der Ansprüche 13 bis 15, worin die nahtlosen Minikapseln mit einem die Freisetzungsgeschwindigkeit kontrollierenden oder magensaftresistenten Überzug überzogen sind, um wirksame Plasmaspiegel des Methylxanthins und des Corticosteroids über eine Dauer von mindestens 12 oder 24 Stunden zu erreichen, in einem Fall ein sofort freisetzender Überzug zusätzlich zum die Freisetzungsgeschwindigkeit kontrollierenden Überzug bereitgestellt wird, in einem Fall der sofort freisetzende Überzug ein Methylxanthin enthält, in einem Fall der sofort freisetzende Überzug ein Corticosteroid enthält, in einem Fall die sofort freisetzende Formulierung nahtlose Minikapseln umfasst, die einen Kern von einem Methylxanthin und/oder einem Corticosteroid, das in einer flüssigen Phase gelöst oder suspendiert oder dispergiert ist, enthalten.

17. Formulierung nach einem der Ansprüche 1 bis 16, die mindestens zwei Gesamtheiten von retardiert freisetzenden Minikapseln, die unterschiedliche in-vitro-Auflösungsprofile haben, umfasst, wobei in einem Fall die Formulierung ein Auflösungsprofil in einem vorgegebenen Medium bereitstellt, sodass etwa 0-25 % des kombinierten Produkts nach 1 Stunde freigesetzt, etwa > 25 % nach 3 Stunden freigesetzt, etwa > 50 % nach 6 Stunden freigesetzt und > 80 % nach 12 Stunden freigesetzt werden, in einem weiteren Fall die Formulierung ein Auflösungsprofil in einem vorgegebenen Medium bereitstellt, sodass < 15 % des kombinierten Produkts nach 1 Stunde freigesetzt werden, etwa > 15 % nach 3 Stunden freigesetzt werden, etwa > 35 % nach 9 Stunden freigesetzt werden, etwa > 45 % nach 12 Stunden freigesetzt werden und > 80 % nach 24 Stunden freigesetzt werden.

18. Formulierung nach einem der Ansprüche 1 bis 17, worin das Methylxanthin aus Theophyllin, Pentoxifyllin und A802715 ausgewählt ist.

19. Formulierung nach einem der Ansprüche 1 bis 18, worin das Corticosteroid aus Dexamethason, Prednisolon, Prednison und Budesonid ausgewählt ist.

20. Formulierung nach einem der Ansprüche 1 bis 19, die eine Kapsel umfasst, die eine Vielzahl von Minikapseln enthält.

21. Formulierung nach Anspruch 20, worin die Kapsel eine weitere Einheit enthält.

22. Formulierung nach einem der Ansprüche 1 bis 19, die eine Kapsel umfasst, die eine Vielzahl von Minikapseln enthält, wobei die Kapsel eine weitere Einheit enthalten kann, die weitere Einheit in einer flüssigen, pulverförmigen, halbfesten, festen oder gasförmigen Form vorliegen kann, die weitere Einheit eine Wirkeinheit umfassen kann.

23. Formulierung nach einem der Ansprüche 1 bis 17, worin mindestens einige der Minikapseln mit einem Bioadhäsivum, wie zum Beispiel einem Mukoadhäsivum, bereitgestellt werden, in einem Fall das Bioadhäsivum von 0 Gewichts-% bis 10 Gewichts-% von einer oder mehreren der folgenden Polymerklassen umfasst: Polyacrylaten, Polyanhydriden, Chitosanen, Carbopolen, Cellulose, Methylcellulose, methylierter Desoxycellulose (m-doc^{®}), Lectinen; in einem weiteren Fall das Bioadhäsivum von 0 Gewichts-% bis 10 Gewichts-% von einem oder mehreren der folgenden thiolierten oder anderweitig derivatisierten Polymeren umfasst: Polyacrylaten, Polyanhydriden, Chitosanen, Carbopolen, Cellulose, Methylcellulose, methylierter Desoxycellulose (m-doc^{®}), Lectinen; in einem Fall das Bioadhäsivum einen Überzug umfasst.

24. Formulierung nach Anspruch 23, worin das Bioadhäsivum in einem Teil oder einer Schicht der Minikapsel eingeschlossen ist, in einem Fall das Bioadhäsivum in einer die Freisetzungsgeschwindigkeit kontrollierenden Schicht eingeschlossen ist, in einem weiteren Fall das Bioadhäsivum in dem verkapselnden Medium eingeschlossen ist.

25. Formulierung nach einem der Ansprüche 1 bis 24, worin mindestens einige der Minikapseln eine Schicht haben, wie zum Beispiel eine äußere Schicht, die in mindestens zwei Teile geteilt ist, in einem Fall die Teile aus der gleichen Zusammensetzung sind, in einem Fall mindestens einige der Teile eine andere Zusammensetzung haben.

26. Formulierung nach einem der Ansprüche 1 bis 25, die Hartgelatinekapseln mit festen, halbfesten oder flüssigen Kernen umfasst.

27. Formulierung nach einem der Ansprüche 1 bis 26, die ein Sachet oder ein Spray oder ein Suppositorium zur analen oder vaginalen Zufiihrung oder intrauterinen Zufiihrung oder ein Suppositorium oder Spray zur bukkalen Zuführung, nasalen Zufiihrung oder pulmonalen Zuführung umfasst.

## Revendications

1. Formulation pharmaceutique comprenant une pluralité de mini-capsules sans soudure ayant un diamètre allant de 0,5 mm à 5 mm, au moins une partie des mini-capsules contenant une méthylxanthine comme l'un des ingrédients actifs, et au moins une partie des mini-capsules contenant un corticostéroïde comme l'autre ingrédient actif.

2. Formulation selon la revendication 1, dans laquelle au moins une partie des mini-capsules contient une méthylxanthine ainsi qu'un corticostéroïde.

3. Formulation selon la revendication 1 ou 2, dans laquelle les mini-capsules comprennent un milieu d'encapsulation, dans l'un des cas le milieu d'encapsulation d'au moins une partie des mini-capsules contenant au moins l'un des ingrédients actifs.

4. Formulation selon l'une quelconque des revendications 1 à 3, dans laquelle au moins une partie des mini-capsules comprend un coeur, dans l'un des cas le coeur contenant au moins un ingrédient actif.

5. Formulation selon l'une quelconque des revendications 1 à 4, dans laquelle au moins une partie des mini-capsules comprend au moins une couche, la couche pouvant contenir au moins un ingrédient actif.

6. Formulation selon la revendication 5, dans laquelle la couche comprend un enrobage destiné à contrôler le moment et/ou le lieu de la libération de l'entité active, dans l'un des cas les mini-capsules sans soudure et enrobées ayant un diamètre allant de 0,5 mm à 5,0 mm, dans l'un des cas les mini-capsules sans soudure et enrobées ayant un diamètre allant de 1,2 mm à 2,0 mm, dans l'un des cas les mini-capsules sans soudure et enrobées ayant un diamètre allant de 1,4 mm à 1,8 mm.

7. Formulation selon la revendication 6, dans laquelle au moins un enrobage est un enrobage à libération immédiate, au moins un enrobage peut être un enrobage à libération progressive, dans l'un des cas l'enrobage comprenant un enrobage à libération progressive et à libération immédiate, dans l'un des cas au moins un enrobage étant un enrobage gastrorésistant.

8. Formulation selon l'une quelconque des revendications 1 à 7, dans laquelle au moins un enrobage est un enrobage bioadhésif tel qu'un enrobage muco-adhésif.

9. Formulation selon l'une quelconque des revendications 5 à 8, dans laquelle la couche comprend une couche tampon.

10. Formulation selon l'une quelconque des revendications 3 à 9, dans laquelle la mini-capsule est formée à partir d'une solution de coeur contenant un ingrédient actif, et une solution d'encapsulation qui forme, lors de son durcissement, le milieu d'encapsulation, dans l'un des cas la solution d'encapsulation contenant un ingrédient actif, dans l'un des cas l'ingrédient actif contenu dans la solution d'encapsulation étant le même que l'ingrédient actif dans la solution de coeur, dans l'un des cas l'ingrédient actif contenu dans la solution d'encapsulation étant différent de l'ingrédient actif dans la solution de coeur, dans l'un des cas l'ingrédient actif contenu dans la solution d'encapsulation étant sous forme de particule micronisée ou nanonisée.

11. Formulation selon l'une quelconque des revendications 3 à 10, dans laquelle la mini-capsule est formée à partir d'une solution contenant le milieu d'encapsulation et un ingrédient actif.

12. Formulation selon l'une quelconque des revendications 1 à 11, comprenant au moins deux populations différentes de mini-capsules, dans l'un des cas l'une des populations de mini-capsules contenant une méthylxanthine et l'autre population de mini-capsules contenant un corticostéroïde, dans l'un des cas environ 50% du produit étant constitué d'une population de mini-capsules sans soudure et à libération progressive d'une méthylxanthine et d'une population d'environ 50% de mini-capsules sans soudure et à libération progressive d'un corticostéroïde, dans l'un des cas le rapport des mini-capsules sans soudure de la méthylxanthine au corticostéroïde étant d'environ 95:5, 90:10, 80:20, 70:30, 60:40, 50:50, 5:95, 10:90, 20:80, 30:70 ou 40:60.

13. Formulation comprenant une méthylxanthine et un corticostéroïde dans une formulation orale multi-particulaire sans soudure.

14. Formulation selon la revendication 13, comprenant des particules à libération progressive ayant un coeur contenant une méthylxanthine et/ou un corticostéroïde dans un solvant ou une phase liquide pharmaceutiquement acceptable encapsulé(e) dans des mini-capsules multi-particulaires sans soudure.

15. Formulation selon la revendication 13, comprenant des particules à libération progressive ayant un coeur contenant une méthylxanthine et/ou un corticostéroïde dans un solvant ou une phase liquide pharmaceutiquement acceptable encapsulé(e) dans des mini-capsules multi-particulaires sans soudure encapsulées d'une enveloppe de gélatine contenant une méthylxanthine et/ou un corticostéroïde.

16. Formulation selon l'une quelconque des revendications 13 à 15, dans laquelle les mini-capsules sans soudure sont enrobées d'un enrobage à cinétique contrôlée ou gastrorésistant afin d'obtenir des taux plasmatiques efficaces de la méthylxanthine et du corticostéroïde sur une période d'au moins 12 ou 24 heures, dans l'un des cas un enrobage à libération immédiate étant fourni en plus de l'enrobage à cinétique contrôlée, dans l'un des cas l'enrobage à libération immédiate contenant une méthylxanthine, dans l'un des cas l'enrobage à libération immédiate contenant un corticostéroïde, dans l'un des cas la formulation à libération immédiate comprenant des mini-capsules sans soudure contenant un coeur d'une méthylxanthine et/ou d'un corticostéroïde solubilisé(e) ou mis(e) en suspension ou dispersé(e) dans une phase liquide.

17. Formulation selon l'une quelconque des revendications 1 à 16, comprenant au moins deux populations de mini-capsules à libération progressive ayant des profils de solubilisation *in vitro* différents, dans l'un des cas la formulation fournissant un profil de solubilisation dans un milieu prédéterminé de façon à ce qu'environ 0-25% du produit combiné soit libéré après 1 heure, environ >25% soit libéré après 3 heures, environ >50% soit libéré après 6 heures et >80% soit libéré après 12 heures, dans un autre cas la formulation fournissant un profil de solubilisation dans un milieu prédéterminé de façon à ce que <15% du produit combiné soit libéré après 1 heure, environ >15% soit libéré après 3 heures, environ >35% soit libéré après 9 heures, environ >45% soit libéré après 12 heures et >80% soit libéré après 24 heures.

18. Formulation selon l'une quelconque des revendications 1 à 17, dans laquelle la méthylxanthine est choisie parmi la théophylline, la pentoxifylline et A802715.

19. Formulation selon l'une quelconque des revendications 1 à 18, dans laquelle le corticostéroïde est choisi parmi la déxaméthasone, la prédnisolone, la prédnisone et le budésonide.

20. Formulation selon l'une quelconque des revendications 1 à 19, comprenant une capsule contenant une pluralité de mini-capsules.

21. Formulation selon la revendication 20, dans laquelle la capsule contient une autre entité.

22. Formulation selon l'une quelconque des revendications 1 à 19, comprenant une capsule contenant une pluralité de mini-capsules, la capsule pouvant contenir une autre entité, l'autre entité pouvant être sous forme liquide, pulvérulente, semi-solide, solide ou gazeuse, l'autre entité pouvant comprendre une entité active.

23. Formulation selon l'une quelconque des revendications 1 à 17, dans laquelle au moins une partie des mini-capsules est accompagnée d'un bioadhésif tel qu'un muco-adhésif, dans l'un des cas le bioadhésif comprenant de 0% à 10% en poids d'une ou plusieurs parmi les classes de polymères suivantes : polyacrylates ; polyanhydrides ; chitosanes ; carbopols ; cellulose ; méthylcellulose ; désoxycellulose méthylée (m-doc^{™}) ; lectines ; dans un autre cas le bioadhésif comprenant de 0% à 10% en poids d'un ou plusieurs parmi les polymères thiolés ou autrement dérivés : polyacrylates ; polyanhydrides ; chitosanes ; carbopols ; cellulose ; méthylcellulose ; désoxycellulose méthylée (m-doc^{™}) ; lectines ; dans un cas le bioadhésif comprenant un enrobage.

24. Formulation selon la revendication 23, dans laquelle le bioadhésif est incorporé dans une partie ou couche de la mini-capsule, dans l'un des cas le bioadhésif étant incorporé dans la couche à cinétique contrôlée, dans un autre cas le bioadhésif étant incorporé dans le milieu d'encapsulation.

25. Formulation selon l'une quelconque des revendications 1 à 24, dans laquelle au moins une partie des mini-capsules possède une couche telle qu'une couche externe qui est divisée en au moins deux parties, dans l'un des cas les parties ayant la même composition, dans l'un des cas au moins certaines parties ayant une composition différente.

26. Formulation selon l'une quelconque des revendications 1 à 25, comprenant des capsules en gélatine dure ayant des coeurs solides, semi-solides ou liquides.

27. Formulation selon l'une quelconque des revendications 1 à 26, comprenant un sachet, ou un saupoudrage, ou un suppositoire destiné à une administration par voie anale ou vaginale ou une administration par voie intra-utérine, ou un suppositoire ou un saupoudrage destiné à une administration par voie buccale, une administration par voie nasale ou une administration par voie pulmonaire.
